# EUROPEAN PATENT SPECIFICATION

(11) **EP 4 023 767 B1**
(45) Date of publication and mention of the grant of the patent: **02.04.2025**
(21) Application number: 20943941.3
(22) Date of filing: 14.10.2020
(51) Int. Cl.: C12Q 1/6848

(54) **METHOD, COMPOSITION AND KIT FOR FLUORESCENT QUANTITATIVE PCR, AND USE THEREOF**
VERFAHREN, ZUSAMMENSETZUNG UND KIT FÜR QUANTITATIVE FLUORESZENZ-PCR UND IHRE VERWENDUNG
PROCÉDÉ, COMPOSITION ET KIT POUR LA PCR QUANTITATIVE PAR FLUORESCENCE, ET LEUR UTILISATION

(30) Priority: 08.07.2020 CN 202010649826
(43) Date of publication of application: 06.07.2022
(73) Proprietor: Sansure Biotech Inc., Changsha, Hunan 410205 (CN)
(72) Inventor: DAI, Lizhong, Changsha High-Tech Industrial Development Zone Hunan 410205 (CN); JI, Bozhi, Changsha High-Tech Industrial Development Zone Hunan 410205 (CN); TAN, Deyong, Changsha High-Tech Industrial Development Zone Hunan 410205 (CN); LIU, Jia, Changsha High-Tech Industrial Development Zone Hunan 410205 (CN); FAN, Xu, Changsha High-Tech Industrial Development Zone Hunan 410205 (CN); DENG, Zhongping, Changsha High-Tech Industrial Development Zone Hunan 410205 (CN)
(74) Representative: Grünecker Patent- und Rechtsanwälte PartG mbB
(86) International application number: PCT/CN2020/120819
(87) International publication number: WO 2022/007224

(56) References cited:
- WO-A1-2011/120049
- WO-A1-2016/168750
- WO-A1-2016/168750
- CN-A- 104 962 608
- CN-A- 107 236 815
- CN-A- 108 300 772
- CN-A- 110 982 876
- CN-A- 111 534 576
- US-A1- 2020 048 682
- XIA HONGYAN ET AL: "Development of single-tube nested real-time PCR assays with long internally quenched probes for detection of norovirus genogroup II", BIOTECHNIQUES, vol. 60, no. 1, 2016, US, pages 28 - 34, XP055922158, ISSN: 0736-6205, Retrieved from the Internet <URL:https://www.future-science.com/doi/pdf/10.2144/000114370> DOI: 10.2144/000114370
- HIROTSU YOSUKE ET AL: "Double-Quencher Probes Improved the Detection Sensitivity of Severe Acute Respiratory Syndrome Coronavirus 2 (SARS-CoV-2) by One-Step RT-PCR", MEDRXIV, 20 March 2020 (2020-03-20), XP055897883, Retrieved from the Internet <URL:https://www.medrxiv.org/content/10.1101/2020.03.17.20037903v1.full.pdf> [retrieved on 20220304], DOI: 10.1101/2020.03.17.20037903

## Description

### Technical Field

The present invention relates to the field of molecular biology detection, and in particular, to the field of fluorescent quantitative PCR detection.

### Background

Nucleic acid Polymerase Chain Reaction (PCR) technology is a molecular biology technology used for amplifying specific nucleic acid fragments (DNA/RNA), so that the number of gene fragments is increased by tens of thousands of times. The core of the principle thereof is that a fluorescent probe primer binds and hybridizes with a complementary sequence in a single-stranded DNA template to form a partial double strand, and DNA synthesis is carried out under the action of DNA polymerase. The binding of the fluorescent probe primer to the single-stranded DNA template is based on the principle of base pairing: base pairing follows the Watson-Crick base pairing principle of G (guanine): C (cytosine), A (adenine): T (thymine)/U (uracil). The principle is widely used in medical diagnosis, scientific research, bioengineering, agriculture and other disciplines. Quantitative Real-time PCR (qPCR) is a method for measuring the total quantity of products after each PCR cycle by using fluorescent chemicals in nucleic acid amplification reactions. It is also a method for quantitative analysis of a specific DNA sequence in a to-be-tested sample by means of an internal or external reference method. qPCR is to perform real-time detection on a PCR process through fluorescent signals during a PCR amplification process. Since there is a linear relationship between the Ct value of the template and the initial copy number of the template in the exponential period of PCR amplification, it becomes the basis for quantification. Fluorescent indicators for qPCR detection are mainly divided into two categories: one is fluorescent probes, such as Taqman probes, and molecular beacon probes; the other is fluorescent dyes that can bind to double-stranded DNA, such as SYBR Green and Eva Green.

As early as in the 1970s, scientist Korana has proposed the idea of nucleic acid amplification in vitro. In 1983, American scientist Kery Mullis got the inspiration for in vitro nucleic acid amplification when he was studying nucleic acid sequencing methods: using test tubes to simulate the natural process of DNA replication in vivo. By providing a series of suitable conditions including template DNA, fluorescent probe primers, DNA polymerase, suitable buffer system, temperature and time for DNA denaturation, rejuvenation and extension, a DNA molecule with known sequences at both ends can be geometrically amplified. This in vitro DNA amplification technology has undergone a series of subsequent optimizations and improvements, and has been successfully applied for the world's first PCR invention patent, US Patent 4683202, in 1987.

Since then, with the use and promotion of PCR technology in clinical and scientific research, subsequent patents have continued to enrich and optimize the details of PCR technology. In 1992, Higuchi et al. first proposed the use of a dynamic PCR method and a closed fluorescence collection approach to detect and analyze the number of target genes, i.e., real-time fluorescent quantitative PCR technology. Real-time fluorescent quantitative PCR analysis is to perform quantitative analysis by detecting the direct correlation between the quantity of amplified products (product labeling fluorescence intensity) and the initial gene quantity. The cycle number Ct value (Cycle threshold) at which the increased fluorescence signal of the amplified product reaches a logarithmic phase has a negative linear relationship with the logarithm of the initial copy number of the template, that is, the number of amplification cycles required to dilute one cup of the initial template to achieve the same logarithmic fluorescence intensity is increased by one cycle (Ct). The increased signal of the amplified product can be displayed by a fluorescent dye of the product DNA, such as Sybr Green I, or detected by a fluorescent probe with a quenching group, such as a Taqman probe. Probes with fluorescent quenching groups include fluorescent labeled probes hydrolyzed by Taq enzyme in qPCR technology and MGB probes that increase binding efficiency on the basis of hydrolyzed fluorescent probes. A variety of subsequent hybridization probe types, such as hybridization probes of a stem-loop structure, two-hybridization (FRET) probes and other novel probes have related uses but all have large limitations in the scope of use, and the sensitivity of these probes still needs to be further enhanced.

In addition, in the application of clinical tests, false negatives are often encountered due to the low sensitivity of the detection method. In particular, the ORF1ab gene of novel coronavirus (2019-nCoV) is easy to be missed due to the low sensitivity of the detection method because of its low expression level.

Therefore, this field requires a method and product that can improve the sensitivity of PCR and reduce the occurrence of false negatives.

CN110982876 relates to a one-step method of directly detecting viral nucleic acid in a sample without the need for extraction, wherein the sample is pretreated with a nucleic acid releasing agent and a qPCR amplification reagent.

WO2016/168750 relates to a method of fluorescent qPCR comprising the use of a fluorescent probe with two quenchers.

### Summary

In view of the above, according to a first aspect, the present invention provides a one - step method for
fluorescent quantitative PCR. The method includes:
1) mixing a sample releasing agent, a sample,
   an upstream and downstream primer pair, a fluorescent probe, and a PCR amplification reagent; and
2) carrying out the fluorescent quantitative PCR,
   where the fluorescent probe has two quenching groups, in which the first quenching group is located at the 3' end and the second quenching group is labeled on the T base and is 10-15 nt apart from the first quenching group, wherein formamide, SDS and a Proclin antibiotic are also mixed in step 1) of the method, wherein the method does not comprise purification and/or extraction steps of nucleic acid.

By means of using the method for fluorescent quantitative PCR of the present invention, background signals in the fluorescent quantitative PCR can be reduced, and furthermore, the sensitivity of PCR is improved, the occurrence of false negative detection is reduced, and the amplification efficiency of the fluorescent quantitative PCR can also be improved.

Without wishing to be bound by theory, a single quenching group does not completely quench the fluorescence of a fluorophore, resulting in a higher background signal. Using the fluorescent probe of the present invention having two quenching groups as defined above further successfully reduces the background signals without affecting the progress of the reaction, thereby improving the sensitivity of PCR, and improving the PCR amplification efficiency.

The additional additives formamide, SDS and a Proclin antibiotic of the present invention can further cooperate with the fluorescent probe having two quenching groups, so that the sensitivity is further improved, and the detection result is more accurate.

In some specific embodiments, the pH value of the additional additive used in the present invention is between 7.0 and 8.8, and more preferably, the pH value of the PCR amplification reagent used in the present invention is 7.5.

In a specific embodiment, the additional additives are Formamide, SDS and Proclin 300, which have a final concentration of 0.01 % to 0.05% (v/v) after being added to a PCR reaction solution.

Furthermore, the length of the fluorescent probe is 18-35 bp, and more preferably, the length of the fluorescent probe is 22-28 bp.

Furthermore, the Tm value of the fluorescent probe is 55-70°C and more preferably, the Tm value of the fluorescent probe is 65-70°C.

Furthermore, the GC content of the fluorescent probe does not exceed 60%.

The quenching groups can be selected from BHQ1, BHQ2, and MGB, but are not limited thereto.

In a specific embodiment, the first quenching group and the second quenching group are the same quenching group. Both the first and second quenching groups can be labeled using a method well known to those skilled in the art.

The fluorescent probe also has a fluorescent group, and the fluorescent group can be selected from FAM, HEX, ROX, VIC, CY5, 5-TAMRA, TET, CY3 and JOE, but is not limited thereto.

The "fluorescent quantitative PCR" mentioned in the present invention is a method for measuring the total quantity of products after each polymerase chain reaction (PCR) cycle by using fluorescent chemicals in nucleic acid amplification reactions. It is also a method for quantitative analysis of a specific nucleic acid sequence in a to-be-tested sample by means of an internal or external reference method.

The "PCR amplification reagent" mentioned in the present invention refers to a reagent used for real-time fluorescent quantitative nucleic acid amplification detection. Those skilled in the art can understand that a qPCR amplification reagent usually contains DNA polymerase, dNTP, a PCR buffer and the like. For example, when the detection object is RNA, the reagent may further include reverse transcriptase. It is not difficult to understand that those skilled in the art can determine the composition and concentration of the PCR amplification reagent according to specific needs (such as, the type and content of the sample).

The "sample releasing agent" mentioned in the present invention refers to a chemical reagent that can release nucleic acid in a sample and can be used for PCR without the need for purification and/or extraction of the nucleic acid. For example, the sample releasing agent is a strongly acidic or basic chemical reagent. An exemplary sample releasing agent may include one or more of 0.01-0.5 mmol/L of surfactin, 100-200 mmol/L of potassium chloride, 50-200 mmol/L of lithium chloride, triethanolamine lauryl sulfate having a mass/volume ratio of 0.1% to 1%, Nonidet P-40 (NP-40) having a volume/volume ratio of 0.1% to 1%, sodium clodecyl sulfonate having a mass/volume ratio of 0.01% to 2%, ethanol having a volume/volume ratio of 0.05% to 1%, and other components. However, the present invention is not limited thereto.

The "one-step method" mentioned in the present invention refers to an Extraction Free Nucleic Acid Release and Amplification Technology (EFNART) of the sample. The technology refers to performing direct sample nucleic acid amplification detection directly by means of a sample nucleic acid releasing agent with strong alkaline properties and a highly compatible amplification system without the need for nucleic acid extraction or purification of the sample.

Using the method of the present invention can improve the sensitivity of the "one-step method", overcome the defects of low sensitivity and inaccurate detection result of the "one-step method", and avoid the occurrence of false negatives. In addition, using the one-step method also saves time and completes the detection of nucleic acid in a very short time, thereby improving the detection efficiency (for example, bringing an accurate diagnosis result and treatment plan to the patient as early as possible opportunity, and playing a key role in hindering the spread of major infectious diseases).

The "sample" mentioned in the present invention refers to a substance containing to-be-tested nucleic acid. The sample may include, but is not limited to, animal and plant cells, bacteria, viruses, fungi, etc., as well as body fluids, tissues, organs, etc. including these.

In particular, the samples mentioned in the present invention are mucus, sputum, pus and urine, etc., and since such samples need to be preserved in a preservation solution, which contains some interfering substances such as antibiotics and surfactants, the sensitivity is usually low when a nucleic acid extraction-free detection method is used.

According to a second aspect, the present invention provides a composition for fluorescent quantitative PCR, including a sample releasing agent, a sample, an upstream and downstream primer pair, a fluorescent probe, and a PCR amplification reagent,
where the fluorescent probe has two quenching groups, in which the first quenching group is located at the 3' end and the second quenching group is labeled on the T base and is 10-15 nt apart from the first quenching group, wherein the composition further comprises formamide, SDS and a Proclin antibiotic.

In some specific embodiments, the pH value of the additional additive used in the composition is between 7.5 and 8.8, and more preferably, the pH value of the PCR amplification reagent used in the present invention is 8.5.

In a specific embodiment, the additional additives are formamide, SDS and Proclin 300, which have
a final concentration of 0.01% to 0.05% (v/v) after being added to a PCR reaction solution.

Furthermore, the length of the fluorescent probe is 18-35 bp, and more preferably, the length of the fluorescent probe is 22-28 bp.

Furthermore, the Tm value of the fluorescent probe is 55-70°C and more preferably, the Tm value of the fluorescent probe is 55-70°C.

Furthermore, the GC content of the fluorescent probe does not exceed 60%.

The quenching groups can be selected from BHQ1, BHQ2, and MGB, but are not limited thereto.

In a specific embodiment, the first quenching group and the second quenching group are the same quenching group.

The fluorescent probe also has a fluorescent group, and the fluorescent group can be selected from FAM, HEX, ROX, VIC, CY5, 5-TAMRA, TET, CY3 and JOE, but is not limited thereto.

According to a third aspect, the present invention provides use of the above-mentioned composition in the preparation of a kit for one-step fluorescent quantitative PCR.

According to a fourth aspect, the present invention provides a kit for one-step fluorescent quantitative PCR. The kit includes the above-mentioned composition.

### Brief Description of the Drawings

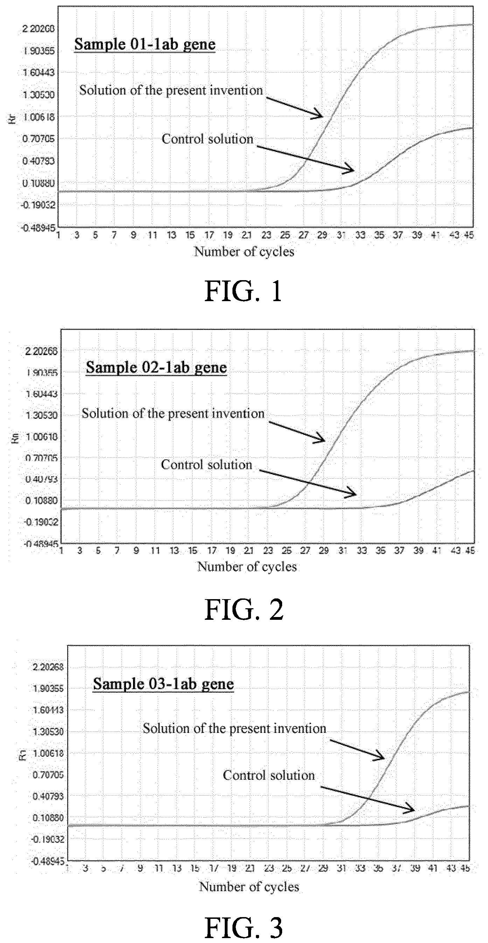

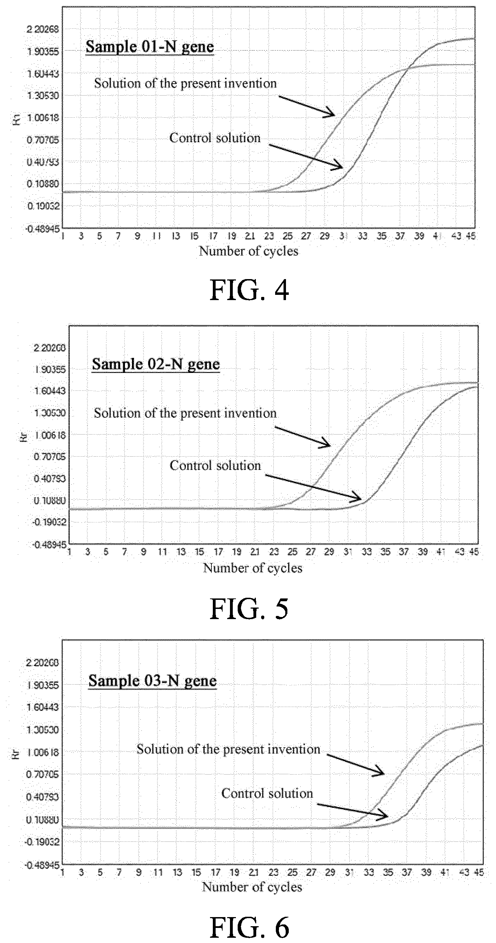

FIG 1 to FIG. 6 show detection results of the composition of the present invention and the compositions of comparative examples for the detection of novel coronavirus (2019-nCoV).

### Detailed Description

Hereinafter, the present invention is described in detail with reference to specific embodiments and examples, and the advantages and various effects of the present invention may be more clearly presented therefrom. It should be understood by those skilled in the art that these specific embodiments and examples are intended to illustrate the present invention, instead of limiting the present invention.

### Example 1. The fluorescent probe having two quenching groups of the present invention improves the sensitivity of PCR

In order to evaluate the application of a double quenching design method of a probe in the present invention, the influence on PCR is investigated. The double quenching design method with the probe of the present invention is compared with a probe having a single quenching group in a PCR amplification reagent. On the premise of not changing a primer sequence and adding a component, the nucleic acid detection of weakly positive samples by different probe designs in the same PCR amplification system is investigated. The comparison method is to use a low-concentration respiratory syncytial virus (RSV) sample that is clinically diagnosed as positive for detection, and the same sample is tested 10 times to verify the positive detection efficiency of the sample. The specific components of each group are shown in Table 1.

**Table 1 Specific components of different probe design solutions**

| | Amplification solution 1 of the present invention | Amplification solution 2 of the present invention | Control amplification solution |
|---|---|---|---|
| Upstream primer sequence | GCAAATATGGAAACATACGTGAACA (SEQ ID NO:1) | | |
| Downstream primer sequence | RGATGAYTGGAACATRGGCACC (SEQ ID NO:2) | | |
| Taqman probe sequence | 5'-FAM- CAGCWGCTGTGTAT( T- BHQ1)GTGGAGCCYT CGTGA-3'-BHQ1 (SEQ ID NO:3) | 5'-FAM- CAGCWGCTGTGTAT GT(T- BHQ1)GGAGCCYTCG TGA-3'-BHQ1 (SEQ ID NO:4) | 5'-FAM- CAGCWGCTGTGTAT GTGGAGCCYTCGTG A-3'-BHQ1 (SEQ ID NO:5) |
| Upstream primer dosage | 10 pmol | | |
| Downstream primer dosage | 10 pmol | | |
| Probe dosage | 5 pmol | | |
| Mg²⁺ | 2 pmol | | |
| dNTPs | 2 pmol | | |
| Enzyme mixture solution | 4 µL | | |
| PCR buffer | 24.65 µL | | |
| Sample dosage | 20 µL | | |
| Total reaction volume | 50 µL | | |

In the above-mentioned comparison experiments, the double quenching design of the probe is investigated, especially the comparison between the design at different base positions and the probe having a single quenching group. The test result shows (Table 2) that it can be seen that the probe designs of amplification solution 1 and amplification solution 2 of the present invention can significantly improve the detection efficiency for low-concentration RSV samples compared with the conventionally designed comparative amplification solution without double quenching. However, compared with the amplification solution 1/2 of the present invention, although a second quenching group is designed on different bases, there is almost no difference in the influence on the amplification detection efficiency, and there is no significant difference in the detection efficiency of the 10 samples. The experimental results of this group show that for the design of two quenching groups on the same probe, the designs at different positions have no significant difference in detection efficiency.

**Table 2 Amplification comparison results of different amplification procedures for 10 low-concentration nucleic acid samples**

| | | | | | | | | | | |
|---|---|---|---|---|---|---|---|---|---|---|
| Amplification solution 1 of the present invention | 35.14 | 35.65 | 34.98 | 36.02 | 36.54 | 3579 | 35.15 | 36.74 | 36.54 | 35.27 |
| Amplification solution 2 of the present invention | 35.15 | 35.31 | 35.69 | 36.46 | 36.93 | 35.82 | 35.25 | 36.47 | 36.75 | 35.46 |
| Control amplification solution | 38.84 | 38.18 | 38.12 | No Ct | No Ct | 37.89 | 37.95 | 38.86 | No Ct | 39.12 |

### Example 2. The fluorescent probe having two quenching groups and additional additives of the present invention improve the sensitivity of PCR

In order to evaluate the application of the double-quenching probe and its PCR amplification reagent components (0.02% of formamide (v/v), 0.03% of SDS (w/v) and 0.01% of Proclin 300 (v/v)) in the present invention, the same probe design principle is employed to compare and modify the fluorescent probe design of respiratory syncytial virus (RSV), and on the premise that the base sequence of the probe remains unchanged, nucleic acid detection of weakly positive samples in the same PCR amplification system is performed using the double quenching group solution of the present invention and the conventional probe design (5'-FAM specific probe sequence-3'-BHQ1). The method of comparison is to use an RSV sample that is clinically diagnosed as positive for gradient dilution step by step, 10-fold dilution (1:9, v/v), 100-fold dilution (1:99, v/v), and 1,000-fold dilution (1:999, v/v), and 10,000-fold dilution (1:9,999, v/v). The detection method is a nucleic acid extraction-free direct amplification detection method of the sample. The method adopted is sample: sample releasing agent: qPCR reaction solution = 10:10:30 (v/v/v) to directly amplify the sample having a total volume of 50 µL. The amplification procedure is shown in Table 3. The specific components of each group are shown in Table 4.

**Table 3 Amplification detection procedure for nucleic acid adopted in the present invention.**

| Step | Temperature | Time | Number of cycles |
|---|---|---|---|
| Reverse transcription | 50°C | 30 min | 1 |
| Pre-denaturation | 95°C | 1 min | 1 |
| Denaturation | 95°C | 15 sec | 40-45 |
| Annealing, extension and fluorescence collection | 60 °C | 30 sec | |

**Table 4 Specific components of different probe design solutions**

| | Amplification solution of the present invention | Control solution |
|---|---|---|
| Upstream primer sequence | GCAAATATGGAAACATACG TGAACA (SEQ ID NO:1) | GCAAATATGGAAAC ATACGTGAACA (SEQ ID NO:1) |
| Downstream primer sequence | RGATGAYTGGAACATRGGC ACC (SEQ ID NO:2) | RGATGAYTGGAACA TRGGCACC (SEQ ID NO:2) |
| Taqman probe sequence | 5'-FAM- CAGCWGCTGTGTAT(T- BHQ1)GTGGAGCCYTCGTG A-3'-BHQ1 (SEQ ID NO:3) | 5'-FAM- CAGCWGCTGTGTAT GTGGAGCCYTCGTG A-3'-BHQ1 (SEQ ID NO:5) |
| Upstream primer dosage | 10 pmol | |
| Downstream primer dosage | 10 pmol | |
| Probe dosage | 5 pmol | |
| Mg²⁺ | 2 pmol | |
| dNTPs | 2 pmol | |
| Enzyme mixture solution | 4 µL | |
| Additive group concentration | 0.03% (v/v) | 0 |
| PCR buffer | 24.65 µL | 24.65 µL |
| Sample dosage | 20 µL | |
| Total reaction | 50 µL | |
| volume | | |

The comparison result in Table 5 shows that for the additive components in the PCR amplification system of the present invention, the sensitivity of the extraction-free amplification of the RSV sample is significantly improved. On the premise that the primer probe sequence and the main components of the PCR amplification system remain unchanged, the optimization of the quenching modification solution of the probe greatly improves the nucleic acid detection capability.

**Table 5 Sensitivity comparison detection solution based on gradient dilution of the sample.**

| | Design solution with the probe of the present invention | Conventional fluorescent probe design solution |
|---|---|---|
| | RSV sample detection Ct value | RSV sample detection Ct value |
| 10-fold dilution of the sample | 29.64 | 31.89 |
| 100-fold dilution of the sample | 32.89 | 35.32 |
| 1,000-fold dilution of the sample | 36.07 | 38.03 |
| 10,000-fold dilution of the sample | 39.46 | No Ct |

### Example 3. The fluorescent probe having two quenching groups and additional additives of the present invention improve the sensitivity of PCR

In order to evaluate the primer probe design solution and the application of additive components (formamide, SDS and Proclin 300) in the present invention, comparative analysis is performed on a reagent component with the primer probe and PCR additive of the present invention and the conventional 3'-end single quenching group (BHQ1) without the primer probe solution of the present invention. The comparison method is to use three novel coronavirus (2019-nCoV) nucleic acid samples (sample 01, sample 02, and sample 03) that are clinically diagnosed as positive to conduct a comparative test in the form of 45 µL of a PCR reaction solution + 5 µL of a nucleic acid sample. The real-time fluorescent quantitative PCR amplification detection procedure is shown in Table 3. The specific components of each group are shown in Table 6.

**Table 6 Specific components of different probe design solutions**

| | Amplification solution of the present invention | Control solution |
|---|---|---|
| ORF 1ab upstream primer sequence | ACAATGCGTTAGCTTACTAC AAC (SEQ ID NO:6) | ACAATGCGTTAGCTT ACTACAAC (SEQ ID NO:6) |
| ORF 1ab downstream primer sequence | TCTAGCCCATTTCAAATCCTG (SEQ ID NO:7) | TCTAGCCCATTTCAA ATCCTG (SEQ ID NO:7) |
| ORF 1ab fluorescent probe sequence | FAM-TAGGTTTGTACT(T- BHQ1)GCATTGTTATCCG- BHQ1 (SEQ ID NO:8) | TAGGTTTGTACTTGC ATTGTTATCCG (SEQ ID NO:9) |
| N gene upstream primer sequence | AGTCCAGATGACCAAATTGGC (SEQ ID NO:10) | |
| N gene downstream primer sequence | ACTGAGATCTTTCATTTTACCGTCAC (SEQ ID NO:11) | |
| N gene fluorescent probe sequence | ACTACCGAAGAGCTACCAGACGA (SEQ ID NO:12) | |
| Upstream primer dosage | 10 pmol | |
| Downstream primer dosage | 10 pmol | |
| Probe dosage | 5 pmol | |
| Mg²⁺ | 2 pmol | |
| dNTPs | 2 pmol | |
| Enzyme mixture solution | 4 µL | |
| Additive group concentration | 0.03% (v/v) | 0 |
| PCR buffer | 33.65 µL | 33.65 µL |
| Nucleic acid dosage | 5 µL | |
| Total reaction volume | 50 µL | |

From the comparison results of amplification in FIG. 1 to FIG. 6 and Table 7, on the premise of using the same nucleic acid template dosage and primer probe dosage, just because of the change of the probe labeling mode and the necessary additive components, between the two, especially the amplification efficiency of the ORF 1ab gene based on a FAM channel is significantly improved between the two. Because the probe modification method of the present invention has obvious advantages in improving the detection sensitivity of nucleic acid reagents.

**Table 7 Comparison results of the amplification effects of the solution of the present invention and a control solution on three nucleic acid samples**

| | PCR reaction solution with additives of the present invention | | Commercial PCR reaction solution | |
|---|---|---|---|---|
| | ORF 1ab gene | N gene | ORF 1ab gene | N gene |
| Sample 01 | 25.05 | 25.20 | 32.92 | 30.10 |
| Sample 02 | 25.35 | 25.45 | 37.91 | 33.17 |
| Sample 03 | 31.88 | 32.19 | 39.69 | 35.24 |

The comparison result in Table 7 shows that for the probe modification and additive components in the present invention, the sensitivity of PCR is significantly improved. The detection capability of the kit is negatively correlated with a Cycle threshold (Ct) value, that is, at the same concentration, the smaller the Ct value is, the higher the detection capability is; the larger the Ct value is, the lower the detection capability is; and No Ct means no amplification. The probe modification and additives in the present invention can significantly improve the nucleic acid detection capability, but for different samples, there are differences between samples in the improvement effect of the PCR additives.

### Example 4. The fluorescent probe having two quenching groups and additional additives of the present invention improve the sensitivity of one-step PCR

In the process of clinical examination application, respiratory samples are highly infectious, such as novel coronavirus, SARS, MERS coronavirus, etc., as well as common influenza A and B viruses, all of which are highly pathogenic. Therefore, completing virus detection in a very short time can bring a diagnosis result and a treatment plan to the patient as early as possible. The solution of the double-quenching probe and the matched PCR amplification additive components in the present invention is also used in the application of rapid sample detection. The main features of the procedure include two major aspects: the two are both directly amplifying the samples without nucleic acid processing, and are respectively: 1. on the premise of no nucleic acid extraction and purification, directly inactivating the collected samples by using a sample releasing agent with an inactivation function, and releasing the nucleic acid in the virus, and using the relevant PCR amplification reagent components for direct amplification detection; and 2. using a very short reverse transcription procedure and a PCR amplification procedure to complete the detection of the virus within 15-35 minutes and giving the detection result. The detection method is a nucleic acid extraction-free direct amplification detection method of the sample. The method adopted is sample: sample releasing agent: qPCR reaction solution = 10:10:30 (v/v/v) to directly amplify the sample having a total volume of 50 µL. In order to compare the detection effects in rapid detection and routine detection, this experimental solution adopts the reaction conditions of the present invention under the rapid amplification procedure and the ordinary amplification procedure, as well as the conventional probe design solution under the conventional amplification procedure to compare the detection efficiency of low-concentration nucleic acid samples and negative samples of novel coronavirus.

**Table 8 One-step PCR and conventional PCR in the solution of the present invention**

| | Conventional amplification procedure | | | Rapid amplification procedure | | |
|---|---|---|---|---|---|---|
| Step | Temperature | Time | Number of cycles | Temperature | Time | Number of cycles |
| Reverse transcription | 50°C | 30 min | 1 | 50°C | 5 min | 1 |
| Pre-denaturation | 95°C | 1 min | 1 | 95°C | 10 sec | 1 |
| Denaturation | 95°C | 15 sec | 40-45 | 95°C | 1 sec | 40-45 |
| Annealing, extension and fluorescence collection | 60 °C | 30 sec | | 60 °C | 1 sec | |

**Table 9 Amplification comparison results of different amplification procedures for 10 low-concentration nucleic acid samples**

| | | | | | | | | | | |
|---|---|---|---|---|---|---|---|---|---|---|
| Solution of the present invention Conventional amplification | 32.42 | 32.46 | 32.59 | 31.76 | 32.77 | 32.92 | 31.83 | 32.74 | 32.09 | 32.40 |
| Solution of the present invention Rapid amplification | 33.15 | 32.84 | 32.16 | 32.56 | 32.98 | 33.23 | 33.75 | 32.47 | 32.95 | 32.63 |
| Control solution Conventional amplification | 35.84 | 36.18 | 36.28 | 35.98 | 36.32 | 35.45 | 36.74 | 36.12 | 36.29 | 36.83 |
| Control solution Rapid amplification | 38.84 | 39.18 | NoCt | NoCt | NoCt | NoCt | 38.74 | 39.42 | NoCt | NoCt |

As can be seen from the detection results of 10 low-concentration samples, all the low-concentration samples are detected using the solution of the present invention. In addition, the detection result obtained using the rapid amplification solution is no different from that using the conventional amplification procedure. Therefore, the experimental results prove that the amplification detection solution based on the double-quenching probe design can be used in both conventional qPCR amplification procedure and fast qPCR amplification procedure, and there is no significant difference between the two different amplification procedures. The solution of the present invention can be used for rapid detection of viruses. In the control solution, the probe design and combination solution without the conventional single quenching group of the present invention are adopted, and in the conventional amplification procedure, all 10 samples are detected, but the amplified Ct value is delayed. However, in the rapid amplification procedure, only four out of the 10 samples are detected to be positive, thus the detection capability is obviously low. Therefore, the comparison result shows that the solution of the present invention can be used for rapid nucleic acid diagnosis of RNA viruses.

### Example 5. The fluorescent probe having two quenching groups and additional additives of the present invention improve the sensitivity of PCR

In order to evaluate the application of the double-quenching probe and its PCR amplification reagent components (Formamide, SDS and Proclin 300) in the present invention, the same probe design principle is employed to compare and modify the fluorescent probe design of respiratory adenovirus (ADV), and on the premise that the base sequence of the probe remains unchanged, nucleic acid detection of weakly positive samples in the same PCR amplification system is performed by comparing the amplification solutions of the double quenching group solution of the present invention and the control solution.

The designs of the four solutions are (as shown in Table 11):
the solution of the present invention: an amplification reagent containing double-quenching fluorescent probe and having additive components
control solution 1: a conventional amplification reagent only containing a double-quenching fluorescent probe
control solution 2: an amplification reagent containing a single-quenching fluorescent probe and having an additive
control solution 3: a conventional amplification reagent only containing a single-quenching fluorescent probe

The method of comparison is to use an ADV sample that is clinically diagnosed as positive for gradient dilution step by step, 10-fold dilution (1:9, v/v), 100-fold dilution (1:99, v/v), and 1,000-fold dilution (1:999, v/v). The detection method is a nucleic acid extraction-free direct amplification detection method of the sample. The method adopted is sample: sample releasing agent: qPCR reaction solution = 10:10:30 (v/v/v) to directly amplify the sample having a total volume of 50 µL. The amplification procedure is shown in Table 10.

**Table 10 Amplification detection procedure for nucleic acid adopted in the present invention.**

| | Conventional amplification procedure | | | Rapid amplification procedure | | |
|---|---|---|---|---|---|---|
| Step | Temperature | Time | Number of cycles | Temperature | Time | Number of cycles |
| Pre-denaturation | 95°C | 1 min | 1 | 95°C | 10 sec | 1 |
| Denaturation | 95°C | 15 sec | 40-45 | 95°C | 3 sec | 40-45 |
| Annealing, extension and fluorescence collection | 60 °C | 30 sec | | 60 °C | 5 sec | |

**Table 11 Specific components of different probe design solutions**

| | Solution of the present invention | Control solution 1 | Control solution 2 | Control solution 3 |
|---|---|---|---|---|
| Upstream primer sequence | TGRAARAGGTAGTTGAGBGTGG (5'-3') (SEQ ID NO:13) | | | |
| Downstream primer sequence | GYAACKCCCAGTTTTTCAACTTYG (5'-3') (SEQ ID NO:14) | | | |
| Probe sequence | 5'-FAM- CYGTGGAYTTCTACGA(BHQ1)R | | 5'-FAM- CYGTGGAYTTCTACGAR | |

| | GCCATGGA-3'-BHQ1 (SEQ ID NO:15) | | GCCATGGA-3'-BHQ1 (SEQ ID NO:15) | |
|---|---|---|---|---|
| Upstream primer dosage | 10 pmol | | | |
| Downstream primer dosage | 10 pmol | | | |
| Probe dosage | 5 pmol | | | |
| Mg²⁺ | 2 pmol | | | |
| dNTPs | 2 pmol | | | |
| Enzyme mixture solution | 4 µL | | | |
| Additive group concentration | 0.03% (v/v) | 0 | 0.03% (v/v) | 0 |
| PCR buffer | 24.65 µL | | | |
| Sample dosage | 20 µL | | | |
| Total reaction volume | 50 µL | | | |

The comparison result in Table 12 shows that there is a significant improvement in the sensitivity of extraction-free amplification of the ADV sample, and the amplification efficiency of the present invention is the best and obviously superior to the solutions based on conventional fluorescent probe design and conventional PCR amplification reagent components.

In addition, the comparison result in Table 12 also proves that the optimization effect of the additives on the two quenching groups is significantly better than that on the single quenching group. In the double quenching group system, the increase of the Ct value of the additive is greater than 1 (having an order of magnitude difference, a difference of 1 in Ct value is equivalent to a difference of one time the template amount), and in the single quenching group system, the increase of the Ct value of the additive is less than 0.5 (essentially equivalent to no increase).

**Table 12 Sensitivity comparison detection solution based on gradient dilution of the sample**

| | Solution of the present invention | Control solution 1 | Control solution 2 | Control solution 3 |
|---|---|---|---|---|
| Conventional qPCR procedure | ADV detection Ct value | | | |
| Sample | 29.64 | 30.89 | 32.14 | 32.65 |
| Rapid amplification procedure | ADV detection Ct value | | | |
| Sample | 29.76 | 30.93 | 32.65 | 33.02 |

## Claims

1. A method for one-step fluorescent quantitative PCR, comprising:
1) mixing a sample releasing agent, an upstream and downstream primer pair, a fluorescent probe, a PCR amplification reagent, and a sample; and
2) carrying out the fluorescent quantitative PCR,
wherein the fluorescent probe has two quenching groups, in which a first quenching group is located at a 3' end and a second quenching group is labeled on a T base and is 10-15 nt apart from the first quenching group,
wherein additional additives are also mixed in step 1) of the method, wherein the additional additives are formamide, SDS, and a Proclin antibiotic, and
wherein the method does not comprise purification and/or extraction steps of nucleic acid.

2. The method according to claim 1, wherein the additional additive Proclin is Proclin 300, and wherein formamide, SDS and Proclin 300 have a final concentration of 0.01% to 0.05% (v/v) after being added to a PCR reaction solution.

3. A composition for extraction-free fluorescent quantitative PCR, comprising a sample releasing agent, an upstream and downstream primer pair, a fluorescent probe, a PCR amplification reagent, and a sample,
wherein the fluorescent probe has two quenching groups, in which a first quenching group is located at a 3' end and a second quenching group is labeled on a T base and is 10-15 nt apart from the first quenching group,
wherein the composition further comprises additional additives, wherein the additional additives are formamide, SDS, and a Proclin antibiotic.

4. The composition according to claim 3, wherein the additional additive Proclin is Proclin 300, and wherein formamide, SDS and Proclin 300 have a final concentration of 0.01% to 0.05% (v/v) after being added to a PCR reaction solution.

5. Use of the composition according to claim 3 or claim 4 in preparation of a kit for one-step fluorescent quantitative PCR.

6. A kit for one-step fluorescent quantitative PCR, wherein the kit comprises the composition according to claim 3 or claim 4.

## Patentansprüche

1. Verfahren für eine quantitative Ein-Schritt-Fluoreszenz-PCR, mit:
1) Mischen eines Probenfreisetzungsmittels, eines vorgeordneten und nachgeordneten Primer-Paars, einer Fluoreszenzsonde, eines PCR-Verstärkungsmittels und einer Probe; und
2) Ausführen der quantitativen Fluoreszenz-PCR,
wobei die Fluoreszenzsonde zwei Abfanggruppen hat, wobei eine erste Abfanggruppe an einem 3'-Ende liegt und eine zweite Abfanggruppe an einer T-Basis markiert ist und 10-15 nt von der ersten Abfanggruppe entfernt ist,
wobei ferner weitere Additive in Schritt 1) des Verfahrens gemischt werden, und wobei die weiteren Additiven Formamid, SDS und ein Proclin-Antibiotikum sind, und
wobei das Verfahren Reinigungs- und/oder Extraktionsschritte für Nukleinsäure nicht enthält.

2. Verfahren nach Anspruch 1, wobei Proclin 300 das weitere Additiv Proclin ist, und wobei Formamid, SDS und Proclin 300 eine finale Konzentration von 0,01 % bis 0,05 % (v/v) nach dem Hinzufügen zu einer PCR-Reaktionslösung haben.

3. Zusammensetzung für extraktionsfreie quantitative Fluoreszenz-PCR mit einem Probenfreisetzungsmittel, einem vorgelagerten und nachgelagerten Primer-Paar, einer Fluoreszenzsonde, einem PCR-Verstärkungsmittel und einer Probe,
wobei die Fluoreszenzsonde zwei Abfanggruppen hat, wobei eine erste Abfanggruppe an einem 3'-Ende liegt und eine zweite Abfanggruppe an einer T-Basis markiert ist und 10-15 nt von der ersten Abfanggruppe entfernt ist,
wobei die Zusammensetzung ferner weitere Additive aufweist und die weiteren Additive Formamid, SDS und ein Proclin-Antibiotikum sind.

4. Zusammensetzung nach Anspruch 3, wobei Proclin 300 das weitere Additiv Proclin ist, und wobei Formamid, SDS und Proclin 300 eine finale Konzentration von 0,01 % bis 0,05 % (v/v) nach dem Hinzufügen zu einer PCR-Reaktionslösung haben.

5. Verwendung der Zusammensetzung nach Anspruch 3 oder Anspruch 4 bei der Herstellung eines Kits für eine quantitative Ein-Schritt-Fluoreszenz-PCR.

6. Kit für eine quantitative Ein-Schritt-Fluoreszenz-PCR, wobei das Kit die Zusammensetzung nach Anspruch 3 oder Anspruch 4 enthält.

## Revendications

1. Procédé de PCR quantitative par fluorescence en une étape, comprenant :
1) le mélange d'un agent de libération d'échantillon, d'une paires d'amorce amont et aval, d'une sonde fluorescente, d'un réactif d'amplification par PCR et d'un échantillon ; et
2) la réalisation de la PCR quantitative par fluorescence,
dans lequel la sonde fluorescente possède deux groupes d'extinction, parmi lesquels un premier groupe d'extinction est situé au niveau d'une extrémité 3' et un second groupe d'extinction est fixé à une base T et se trouve de 10 à 15 nt de distance du premier groupe d'extinction,
dans lequel des additifs supplémentaires sont également mélangés à l'étape 1) du procédé, dans lequel les additifs supplémentaires sont le formamide, le SDS et un antibiotique Proclin, et
dans lequel le procédé ne comprend pas d'étapes de purification et/ou d'extraction d'acide nucléique.

2. Procédé selon la revendication 1, dans lequel l'additif supplémentaire Proclin est le Proclin 300, et dans lequel le formamide, le SDS et le Proclin 300 ont une concentration finale de 0,01 % à 0,05 % (v/v) après avoir été ajoutés à une solution de réaction PCR.

3. Composition pour de la PCR quantitative par fluorescence sans extraction, comprenant un agent de libération d'échantillon, une paires d'amorce amont et aval, une sonde fluorescente, un réactif d'amplification par PCR et un échantillon,
dans laquelle la sonde fluorescente possède deux groupes d'extinction, parmi lesquels un premier groupe d'extinction est situé au niveau d'une extrémité 3' et un second groupe d'extinction est fixé à une base T et se trouve de 10 à 15 nt de distance du premier groupe d'extinction,
dans laquelle la composition comprend, en outre, des additifs supplémentaires, dans laquelle les additifs supplémentaires sont le formamide, le SDS et un antibiotique Proclin.

4. Composition selon la revendication 3, dans laquelle l'additif supplémentaire Proclin est le Proclin 300, et dans laquelle le formamide, le SDS et le Proclin 300 ont une concentration finale de 0,01 % à 0,05 % (v/v) après avoir été ajoutés à une solution de réaction PCR.

5. Utilisation de la composition selon la revendication 3 ou la revendication 4 dans la préparation d'une trousse de PCR quantitative par fluorescence en une étape.

6. Trousse de PCR quantitative par fluorescence en une étape, dans laquelle la trousse comprend la composition selon la revendication 3 ou la revendication 4.
